Europäisches Patentamt

European Patent Office

Office européen des brevets

(11) Publication number: **0 048 148**
**B1**

## EUROPEAN PATENT SPECIFICATION

(45) Date of publication of patent specification: **11.09.85**

(51) Int. Cl.⁴: **C 02 F 3/28, C 02 F 11/04**

(21) Application number: **81304184.5**

(22) Date of filing: **11.09.81**

(54) Process of and digester for anaerobic treatment of waste water.

(30) Priority: **15.09.80 US 187470**
**15.09.80 US 187551**

(43) Date of publication of application:
**24.03.82 Bulletin 82/12**

(45) Publication of the grant of the patent:
**11.09.85 Bulletin 85/37**

(84) Designated Contracting States:
**AT BE CH DE FR GB IT LI LU NL SE**

(56) References cited:
**EP-A-0 012 658**
**GB-A-1 557 282**
**GB-A-1 567 578**
**US-A-3 829 377**
**US-A-4 211 647**

(73) Proprietor: **BACARDI CORPORATION**
**San Juan**
**Puerto Rico (US)**

(72) Inventor: **Benjes, Henry H.**
**10230 Lamar**
**Overland Park Kansas 66207 (US)**
Inventor: **Wahbeh, Valery N.**
**9629 Lee Blvd.**
**Leawood Kansas 66206 (US)**
Inventor: **Stukenberg, John R.**
**10127 Mastin**
**Overland Park Kansas 66212 (US)**

(74) Representative: **Williams, Trevor John et al**
**J.A. KEMP & CO. 14 South Square Gray's Inn**
**London WC1R 5EU (GB)**

Courier Press, Leamington Spa, England.

## Description

The present invention relates to treatment of waste water or sewage so as to remove impurities therefrom, in particular to the removal of oxygen demanding impurities and the production of methane therefrom.

Historically there have been a great number of devices and processes for removing impurities from the waste water so as to improve the quality thereof. Such devices have included clarifiers and digesters, and such processes have included both aerobic and anaerobic processes, examples of which are commonly referred to as filter and contact processes. In a typical filter process the waste water is directed upwardly through a filter vessel with microorganisms therein and is thereafter passed through a degasifier and into a settling basin. An underflow from the settling basin is then returned to the filter vessel. In the contact process waste water is injected into a vessel and agitated in contact with sludge containing microorganisms. Effluent leaving the contact vessel is passed through a degasifier and into a settling tank. Heavier sludge falls to the bottom of the settling tank and is recirculated to the contact vessel.

A typical prior filter process is shown in GB—A—1567578 as comprising a process for the anaerobic treatment of waste water to remove oxygen demanding material therefrom by activity of microorganisms on said waste water within an anaerobic digester containing methane producing microorganisms in the media, wherein the waste water is fed to the media which has a high surface area carrying a proportion of the microorganisms, the waste water is flowed through the media, a substantial portion of the waste water which has passed through the media is continuously recirculated back into the digester for repeated flowing through said media, the volume recirculated being from one to ten times the volume of the influence stream; and an effluent stream is produced from the digester after substantial removal of the oxygen demanding material from the waste water by the microorganisms with methane being produced in the digester.

There are a large number of industries which, because of the nature of the product thereof, produce a waste water solution which is very high in organic constituents and is generally unacceptable for disposition in a lake, a river or the like because of the high oxygen demand associated therewith. In particularly, environmental laws and regulations are becomming increasingly stringent in requiring the removal of chemical and biological oxygen demanding substances (COD and BOD) from waste water before such water is allowed to flow into public waterways. An example of such waste water is the effluent from processes wherein molasses or the like is fermented and distilled in the production of the alcoholic beverages or ethanol for the use in gasohol, such waste being commonly referred to as slops or mostos. The mostos, which is substantially underflow produced in the distillation process, typically has certain elements such as yeast already removed therefrom; however, same will typically have a very high oxygen demand.

While a number of the prior art processes are in various degrees successful in removing a portion of the oxygen demand from waste water, such as mostos, such processes typically require a degasifier and settler along with an anaerobic vessel with resultant complexities and problems associated with multiple pieces of equipment. Also, prior art processes frequently require both anaerobic and aerobic stages. In many of the prior art processes the methane normally produced during the reduction of oxygen demanding components is lost to the atmosphere.

It has been found that by the addition of certain features to an anaerobic digester, the ancillary equipment such as degasifiers and clarifiers can be substantially eliminated while increasing the load passing therethrough with substantially equivalent reduction in oxygen demanding components as compared to prior art devices and methods.

The present invention is characterized in that the waste water is distributed near an upper portion of the media and is flowed downwardly through the media; the recirculated water is continuously recirculated directly from the bottom to the top of the digester carrying microorganisms for dispersion in the top of the media, the recirculation waste water being abstracted at a plurality of separate outlets (45) at the bottom of the digester and recirculated through a plurality of routes (47) to be discharged at a plurality of spaced locations into the top of the digester; in that an effluent stream is withdrawn directly from the digester as a minor portion of the fluid flowing within said digester; and in that a portion of the methane produced in the digester is removed therefrom separately from the effluent stream. With this arrangement the direct recirculation of the waste water through a plurality of filters together with a microbiological material carried thereby from the media ensures that the waste water averages a number of passes through the treatment media with the media being kept automatically in a highly efficient condition without areas of stagnation such that the waste water very highly loaded with oxygen demanding organic material is efficiently and quickly brought into a substantially oxygen-free state ready for discharge.

While it has been proposed in GB—A—1557282 to provide a filter process type apparatus in which the flow of waste water may be either upwardly or downwardly through the filter and in which there is some degree of recirculation, this prior document is solely concerned with obtaining drinking water from only a slightly contaminated source of water such that only a minimal degree of treatment is required. As a consequence there is no appreciation of the benefits which can be obtained in a large volume treatment plant from downward as opposed to upward flow with recirculation and also the type of filter used with separate floating bodies shows that there can necessarily only be a gentle downward flow which is to be expected in view of the direct discharge of treated water. This prior document makes it clear that it is clean treated water which is recirculated simply to maintain minimal flow conditions and contains no suggestion of the benefits to be obtained from the recirculation carrying released biomass back directly into the top of the digester with the process of the present invention requiring the provision of recirculation means separate from the treated effluent discharge means.

By having the recirculation of waste water abstracted at a plurality of separate outlets at the bottom of the digester and recirculated through separate routes to discharge, at different locations, into the top of the digester there is provision for the proper distribution of flow patterns throughout the media such that full and efficient use can be obtained of the biomass loaded media.

According to a further aspect of the invention there is provided a digester having an inlet and an outlet and adapted in use to have microorganisms carried by a high surface area porous structural media therein for interaction with a fluid flowing through the digester from an inlet to an outlet, characterized in that the inlet is positioned in an upper portion of the digester and the outlet opens from a lower portion of the digester such that the fluid is urged to flow downwardly through the digester during use thereof; and in that separate recirculation devices are provided for continuously recirculating fluid from the lower portion of the digester directly to the upper portion thereof such that the fluid flow in the digester during use thereof is downwardly therethrough and containing microorganisms and oxygen demanding organic material to be returned to the upper portion of the digester, said separate recirculation devices being arranged individually to abstract waste water from spaced locations at the bottom of the digester and return it to different respective locations at the top of the digester, the apparatus being dimensioned with the recirculation devices being together adapted in use of the digester to recirculate a flow of liquid of one to ten times the flow of influent liquid through said inlet.

The digester, in use, contains a microorganism which preferentially produces methane when in contact with contaminant material in the waste water and reduces the oxygen demand of such material. Also preferentially, the microorganisms tend to propagate on the media surface and engage the waste water flowing therethrough. Nitrogen and phosphorous containing materials. and/or other nutrients are added as necessary to promote growth of the microorganisms. As the waste water flows downwardly through the digester, methane is released therefrom which bubbles to the surface whereupon the methane is collected for combusting to produce energy. The methane bubbles tend to scour and agitate microorganism biomass on the media. A buffering component may be added to the digester or influent waste water so as to produce an optimum pH for the process. Preferred temperatures of the digesting process are 95°F (35°C) and the range of 125° to 130°F (52° to 54°C). During the process a portion of the microorganism biomass growing on the media surface is sloughed therefrom and settles to the floor of the digester. The sloughed biomass along with a portion of the waste water is recirculated from the bottom of the digester to the top thereof, preferably at a rate of five times the flow of the influent to the digester. An effluent flow is removed from the digester through a stand pipe with an associated trap to prevent atmospheric air from entering the digester and methane from escaping into the atmosphere. Additional treatment may be later applied to effluent if desired. Where necessary to remove biomass from the filter media and to agitate the biomass so as to better mix with the waste water, the methane stream is selectively recirculated into the bottom of the digester and bubbles up through the media and the waste water therein. Preferably a pressure of approximately one pound per square inch (0.07 kilo/sq.cm) is maintained in the digester to urge carbon dioxide produced during the process to remain in solution with the effluent.

The present invention will be further described, by way of example, with reference to the accompanying drawings, wherein:

Figure 1 is a schematic diagram of an apparatus performing the process of the present invention including a digester;

Figure 2 is a top plan view of the digester including piping associated therewith;

Figure 3 is a cross-sectional view of the digester taken along line 3—3 of Figure 2;

Figure 4 is a top plan view of the digester with portions broken away at various levels to show details thereof;

Figure 5 is a fragmentary and enlarged vertical cross-sectional view of the digester showing a side thereof with an associated overflow; and

Figure 6 is a fragmentary horizontal cross-sectional view of the digester showing the overflow as seen in Figure 5.

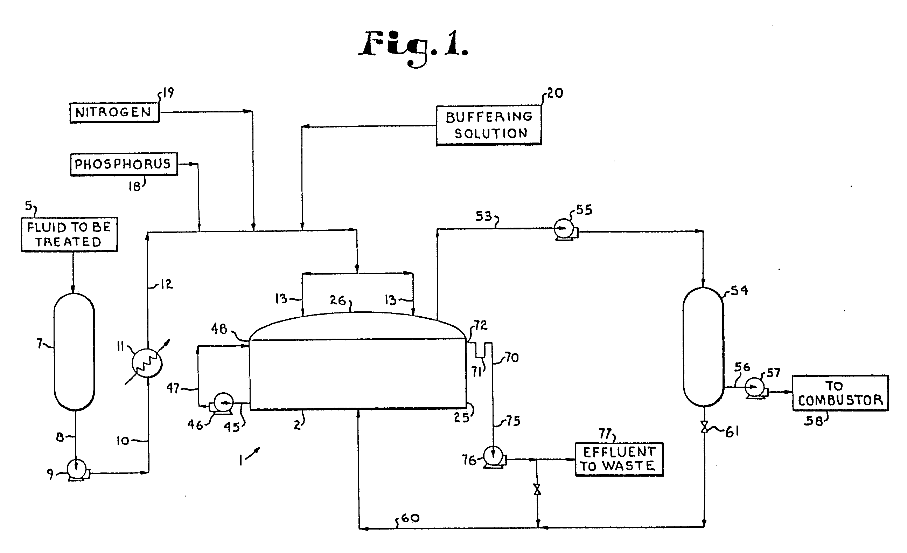

An apparatus 1 for treatment of waste water is schematically illustrated in Figure 1 as comprising an anaerobic digester 2.

A source 5 is shown of fluid to be treated by the apparatus 1. The fluid may be any waste water solution or the like having organic and/or other contaminants suitable for being acted upon by microorganisms to reduce the amount of organic contamination, especially elements creating an oxygen demand therein. In particular, in the presently described embodiment this fluid is a waste stream of a molasses fermentation and distillation process comprising the non-distilled portion of the fermented molasses, especially carbohydrate residue, less the yeast contained therein. This fluid may also contain other materials such as barrel washing and the like and is generally relatively high in organic substances having a biological and chemical oxygen demand (BOD and COD). The fluid exits the fermentation process at a temperature in the nature of 200°F (93°C) and enters a holding tank 7. The holding tank 7 is preferably heat insulated and equipped with agitators (not shown). The fluid from the holding tank 7 is selectively allowed to flow through a conduit 8 and into a pump 9 which is a constant volume per unit time pump and thereafter through a conduit 10 under pressure from the pump 9. The fluid then passes through a heat exchanger 11 which may be selectively cooled by a flow stream of fermented molasses prior to distillation thereof or by cooling water from a conventional cooling tower. The fluid may also be selectively heated by the heat exchanger 11. The fluid then passes through the conduit 12 into the digester 2 via four distribution conduits 13 which enter the digester 2 at somewhat symmetrically spaced locations.

The box 18 generally represents input of phosphorous in a usable form for growth development of microorganisms of the anaerobic methane producing or chemically reducing type. A suitable phosphorous form would be diammonium phosphate as fed to the process by a conventional system well-known in the art. The box 19 generally represents input of nitrogen into the fluid stream which nitrogen is also useful for the growth and development of microorganisms of the type used in the digester 2. A suitable source for the nitrogen may be anhydrous ammonia as provided by a conventional ammonia handling system or alternatively, by use of the ammonium phosphate or the like. It is noted that some fluids may contain a suitable source of phosphorous and nitrogen and thus supplementation thereof will not be necessary.

Preferably a buffering solution is added to the fluid before entry thereof into the digester so that the pH of the fluid entering the digester is in the range of approximately 5 to 9 and preferably between 6.5 and 7.0. As the pH of mostos is approximately 4.5, a basic buffering solution is normally added thereto and the supply of the buffering solution is represented by the box 20. The buffering solution should be able to bring the pH of the fluid to the preferred range and maintain same in that range while the fluid is being acted upon by microorganisms in the digester. In particular, bacteria and other microorganisms present in digesters typically first convert the organic material in the fluid stream to an organic acid which, if there is no buffering solution present, will substantially lower the pH of the fluid in the digester. A suitable buffering solution would be caustic soda (sodium hydroxide) or lime, although numerous similar substances may be utilized providing same are compatible with growth of microorganisms in the digester 2. Although not shown, where HS ion toxicity in the digester 2 is a problem, ferric chloride or the like may also be added with the buffering solution.

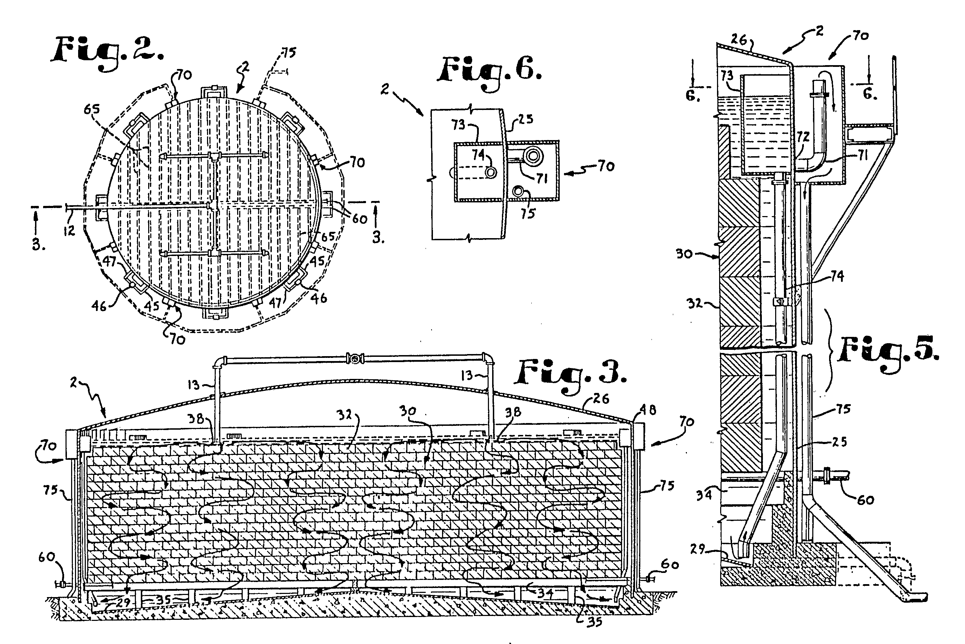

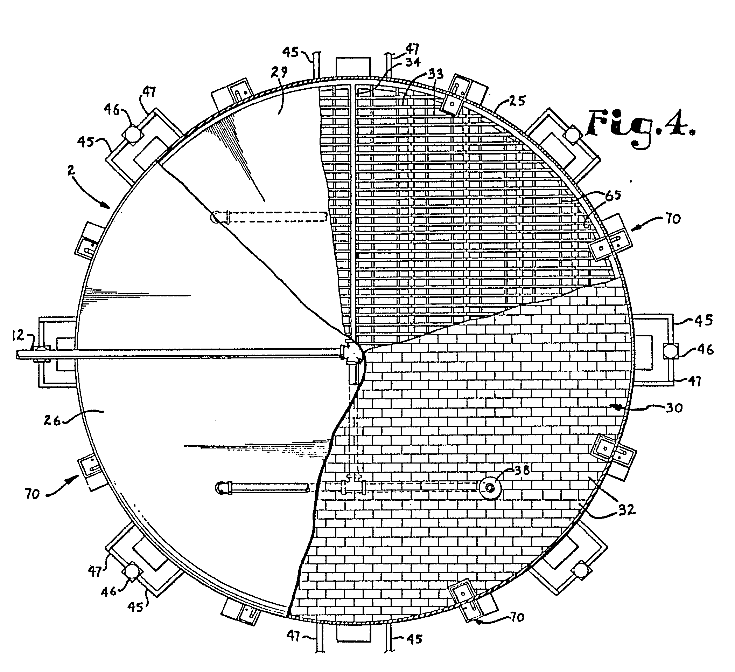

The digester 2 is a large cylindrical vessel 25 having a free standing dome 26 which is substantially air-tight to prevent air from entering the digester 2 and destroying the anaerobic or oxygen-free state therein and to prevent escape of methane to the atmosphere. In the illustrated embodiment, as seen in Figure 3, the floor 29 of the digester 2 is raised at the center thereof and slopes gradually downwardly toward the outer wall thereof. The interior of the vessel preferably contains a lattice, matrix or other suitable media 30 having a substantial surface area thereon. Media 30 should be suitable for retaining and allowing growth of at least a portion of the microorganisms thereon, such as conventional packing. However, it is important that the flow of the fluid through the digester be somewhat tortuous so as to encourage the fluid to engage and mix with the microorganisms, yet not be impeded. In particular, the path and size of the openings in the media should be of sufficient porosity to allow microorganism biomass which is sloughed from the media 30 to fall or settle to a lower portion of the digester 2 and not to plug the flow pathway. Media 30 having openings therein of approximately one inch (2.5 cms) or slightly greater are considered sufficient. One alternative media 30 would be blocks or bundles 32 of PVC plastics such as sold under the trademarks Vinyl Core and Koro-Z, that is corrugated polyvinyl chloride assembled into self supporting modules, as manufactured by the B. F. Goodrich Company. In the illustrated embodiment, the bundles 32 of the media 30 substantially fill more than the upper two thirds of the digester 2 up to near the dome 26. The bundles 32 are supported upon cross-members 33 and 34 which are in turn supported by upright stanchions 35 resting upon the floor 29 of the digester 2. There is a substantially open space between the media 30 and the floor 29 such that microorganism biomass sloughed from the media 30 can fall to the floor 29 and be urged outwardly toward the side wall of the digester 2 without interference from the media 30. The fluid entering the digester 2 through the conduits 13 is preferably evenly distributed by a plurality of radially spaced and horizontally directing nozzles 38 above the media 30.

Recirculation means comprising pumps and conduits recirculate fluid from near the floor 29 of the digester 2 to near the top of the media 30. In particular, in the illustrated embodiment, a plurality of radially spaced conduits 45 are positioned near the lower end of the side wall of the digester 2 and provide for flow of fluid including microorganism biomass or sludge therefrom to an associated pump 46. The pump 46 recirculates flow through the conduit 47 back into the digester 2 near the top 48 thereof. In the illustrated embodiment there are eight recirculation paths including the conduits 45, 47 and pumps 46. It is desirable continually and directly to recirculate fluid and the microorganism biomass associated therewith from the bottom of the digester 2 to the top thereof such that the biomass is more likely to engage and interact with the digester influent fluid. Such recirculation is also believed to reduce the amount of acidity normally formed near the location where the influent enters the digester 2, as the methane forming bacteria in the biomass can act more quickly. Although the optimum recirculation rate varies substantially between types of fluids and various conditions in the digester 2, a suitable recirculation rate for the presently described process is found to be approximately five times the flow rate of the influent fluid to the digester 2.

The microorganism utilized in the process may be any microorganism which will convert organic material to methane under anaerobic conditions and the other conditions present within the digester 2. A suitable culture of such microorganisms may be obtained by removing a seed or starter culture from a conventional digester or simply by including a sample of fresh cow manure in the digester during start-up period.

Methane produced within the digester 2 bubbles to the surface of the fluid therein and is collected within the dome 26 and is conducted therefrom by conduit 53 to a holding tank 54 *via* a compressor or pump 55. The methane is normally taken from the holding tank 54 through conduit 56 and pressurized by pump 57 after passage through a desulfizer when necessary. Such methane is delivered to a combuster represented by the box 58 which may be a boiler for producing steam or a heater for distilling the fermented molasses. It may also be desirable at times to urge a portion of the microorganism biomass from the media 30 as the biomass grows sufficiently large to occlude the passageways through the media 30 or to agitate the biomass within the digester 2. When such occlusion occurs, or when additional scouring of the media 30 is desired, the methane in the holding tank 54 may be selectively routed by operation of valve 61 through conduit 60 under pressure so as to recirculate and enter a lower portion of the digester 2 and be sparged therein by nozzles or apertures along distribution lines 65. The sparged methane tends to loosen, stir and agitate the biomass and urge same to mix or interact with fluid in the digester 2 when the normal rise of methane gas through the media 30 is not sufficient to do so, especially in the vicinity of the digester floor 29.

Although there is a wide range of temperatures within which methane producing microorganisms will function and any of these temperatures are suitable for operation of the present invention, it has been found in the present process that there are two preferred temperature states. The first such temperature state is normally referred to as the mesophilic range around 95°F (35°C) and the other is the thermophilic range between approximately 125° to 130°F (52° to 54°C). Normally the latter range will provide faster methane production and will be used herein since the waste water treated will already be relatively warm.

The digester 2 is also provided with an effluent outflow device 70. The device 70 includes a liquid trap 71 attached to an upper end 72 of the fluid containing portion of the digester 2. Interior of the wall of the digester 2 and communicating with the trap 71 is an open top collection box 73 which in turn communicates with a conduit 74. While the effluent may be withdrawn from anywhere along the vessel side 25, the inlet to the conduit 74, which is the normal location of exit of the effluent from the digester 2, is near the digester bottom 29 and near wall 25. The trap 71 overflows into the conduit 75 and thereafter is transferred by the pump 76 or gravity. In the illustrated embodiment the effluent is wasted to a sewer or the like as represented by box 77.

Thus, in the present embodiment fluid flows into the digester 2 near the top thereof, flows downwardly through the media 30, is recirculated by the recirculating means directly from near the bottom of the digester 2 to near the top thereof at a rate substantially greater than the influent rate, and is removed from the digester 2 after the treatment by the microorganisms to reduce oxygen demand in the effluent and to produce methane.

Preferably, a slight superatmospheric pressure is maintained within the digester 2. This slight pressure urges carbon dioxide which is also produced by the methane producing microorganisms to remain in solution in the fluid within the digester 2. Thus, the carbon dioxide tends to leave the digester in the effluent stream rather than in the methane stream. Too great a pressure within the digester 2 is normally avoided due to problems of maintaining seals about the effluent traps 71 but may be utilized when necessary to further reduce carbon dioxide in the methane. A suitable pressure has been found to be in the nature of one pound per square inch (0.07 kilo/sq.cm) which is estimated to provide a gas mixture to the holding tank 54 in the nature of approximately 60 to 70 percent methane with substantially the remainder being carbon dioxide. A wider range of pressures may be utilized and it is foreseen that the pressure could even be a slight vacuum within the digester 2.

In use the method of utilizing the apparatus 1 comprises distributing the influent fluid to be treated near the top of the digester 2, flowing the fluid downwardly through the media 30 so as to contact microorganisms attached to and in the vicinity of such media, recirculating a portion of the fluid from a lower portion of the digester 2 beneath the media 30 directly to an upper portion of the digester 2 near the location of the distribution of the influent therein, and removing an effluent portion of the fluid from the digester 2, such effluent having been substantially treated by the microorganisms so as to remove methane therefrom and to reduce the biological oxygen demand thereof.

Preferably, the effluent into the digester 2 is buffered so as to maintain a pH in the nature of 6.5 to 7 with the effluent of the digester desired to be within the range of pH of 6 to 9. Nutrients are added to the influent to optimize growth of the bacteria in the digester 2. Also preferably the digester is maintained at a temperature of optimum growth and therefore activity by the microorganisms. Although such temperature varies somewhat, a suitable temperature has been found to be 95°F (35°C) or alternatively within the range of 125° to 130°F (52°C to 54°C). The pressure of the digester is also maintained to optimize retention of carbon dioxide within the effluent from the digester 2, while still allowing function of the effluent trap 71. Gas from the methane holding tank 54 is diverted to the bottom of the digester 2 and bubbled up therethrough, when it is desired to remove additional biomass from the media 30 or to agitate the biomass to improve interaction with the fluid in the digester 2.

It is foreseen that additional treatment may be provided for the effluent of the digester 2 to remove additional contaminants from the waste water. It is also foreseen that the effluent or another liquid could be recycled under high pressure to the bottom of the digester to agitate the biomass and fluid therein instead of the methane.

The following examples are included for purpose of illustration only and are not intended to be limiting with reference to the present invention.

Example I

The following is a calculated example of the effects of the process disclosed in the preferred embodiment:

| | | |
|---|---|---|
| Influent flow rate of mostos to digester in gallons per day (litres per day) | 340,000 | (1,290,000) |
| BOD in grams per litre | 47 | |
| COD in grams per litre | 92 | |
| Pressure in digester in pounds per square inch (kilos/sq.cm) | 1 | (0.07) |
| pH of mostos before process | 4.5 | |
| pH of mostos after addition of NaOH | 7.0 | |
| Temperature in digester | 128°F | (53°C) |
| Height of digester in feet at side (in metres) | 40 | (12) |
| Diameter of digester in feet (metres) | 120 | (37) |
| Loading of BOD on media in pounds of BOD per 1000 cubic feet of media (kilos/m³) | 400 | (6.4) |
| Dibasic Ammonium Phosphate addition in pounds per day (kilos per day) | 437 | (198) |
| Recirculation rate with respect to flow of influent | 5 | |
| Loading of fluid at top of media (influent plus recirculation) in gallons per minute per square foot (litres/min/m²) | 0.13 | (5.29) |
| Methane production in cubic feet (metres³) | 1,000,000 | (28,000) |
| COD in effluent in grams per litre | 32 | |
| BOD in effluent in grams per litre | 14.1 | |
| Biological solids in effluent in pounds per day (kilos per day) | 16,000 | (7,260) |

Example II

A tank holding 2,300 gallons (8,700 litres) of fluid when substantially filled with media is maintained at 98°F (37°C) and a pH in the range of 5 to 8, preferably 6.5 to 7.5. Mostos is added at a rate in the range of 115 to 460 gallons per day (435 to 1740 litres per day) after neutralization by lime, sodium carbonate, sodium bicarbonate and/or sodium hydroxide. Nutrients containing nitrogen and phosphorous from a suitable source are added at a ratio of 100 parts organic carbon to 5 parts nitrogen to 1 part phosphorous. Recirculation is in a range of from 1 to 10 times the rate of addition of mostos to the tank. Influent BOD is 25,000 to 45,000 parts per million (ppm) and COD is 70,000 to 110,000 ppm. Retention time varies in the range of 5 to 20 days, preferably 7 days. It is calculated that BOD removal is 60 to 95 percent, COD removal is 60 to 90 percent, and methane gas generation is 4 to 10 cubic feet per pound (250 to 624 litres per kilo) of COD removed.

Example III

The same conditions and results as in Example II except temperature is maintained in the range of 120° to 130°F (49° to 54°C). and retention time is generally from 2 to 20 days, preferably about 5 days.

**Claims**

1. A process for the anaerobic treatment of waste water to remove oxygen demanding material therefrom by activity of microorganisms on said waste water within an anaerobic digester containing methane producing microorganisms in the media, wherein the waste water is fed to the media (30) which has a high surface area carrying a proportion of the microorganisms, the waste water is flowed through the media, a substantial portion of the waste water which has passed through the media is continuously recirculated back into the digester for repeated flowing through said media, the volume recirculated being from one to ten times the volume of the influent stream, and an effluent stream is produced from the digester after substantial removal of the oxygen demanding material from the waste water by the microorganisms with methane being produced in the digester; characterized in that the waste water is distributed (at 38) near an upper portion of the media (30) and is flowed downwardly through the media; the recirculated water is recirculated directly from the bottom to the top of the digester carrying microorganisms for digestion in the top of the media, the recirculation waste water being abstracted at a plurality of separate outlets (45) at the bottom of the digester and recirculated through a plurality of routes (47) to be discharged at a plurality of spaced locations into the top of the digester; in that an effluent stream is withdrawn directly from the digester as a minor portion of the fluid flowing within the digester; and in that a portion of the methane produced in the digester is removed therefrom separately from the effluent stream.

2. A process according to claim 1, characterized in that the recirculation is such that the volume recirculated is approximately five times the volume of the influent stream.

3. A process according to claim 1 or 2, characterized in that the methane is removed from the top of the digester and a portion of the methane is returned to the lower portion of the digester and allowed to bubble upwardly through the digester so as to pass through said media.

4. A process according to claim 1, 2 or 3 characterized in that nutrients are added to said influent so as to maximize growth of said microorganisms; the temperature within the digester is maintained between approximately 35° and 54°C.; and said influent is buffered to maintain a pH approximately within the range of 6.5 to 7 in the digester.

5. A process according to claim 4, characterized in that the nutrients are biological suppliers of nitrogen and phosphorous, said temperature being within the range of 52° to 54°C.

6. A process according to any preceding claim, characterized in that said effluent stream is substantially mostos from a molasses fermentation and distillation process.

7. A process according to any preceding claim, characterized in that the methane is collected and combusted so as to provide usable energy.

8. A digester having an inlet and an outlet and adapted in use to have microorganisms carried by a high surface area porous structural media therein for interaction with a fluid flowing through the digester (2) from an inlet (38) to an outlet (74) characterized in that the inlet (38) is positioned in an upper portion of the digester (2) and the outlet (74) opens from a lower portion of the digester such that the fluid is urged to flow downwardly through the digester during use thereof; in that separate recirculation devices (45, 46, 47) are provided for continuously recirculating fluid from the lower portion of the digester directly to the upper portion thereof such that the fluid flow in the digester during use thereof is downwardly therethrough and containing microorganisms and oxygen demanding organic material to be returned to the upper portion of the digester, said separate recirculation devices being arranged individually to abstract waste water from spaced locations at the bottom of the digester and return it to different respective locations at the top of the digester, the apparatus being dimensioned with the recirculation devices being together adapted in use of the digester to recirculate a flow of liquid of one to ten times the flow of influent liquid through said inlet.

9. A digester according to claim 8, characterized in that each of the recirculation devices comprises a pump (46) having a suction conduit (45) communicating with the lower portion of the digester.

10. A digester according to claim 9, characterized in that the said suction conduits (45) are substantially equally spaced circumferentially about the digester lower portion.

11. A digester according to claim 8, 9 or 10, characterized in that distribution means (60) is provided adapted for distributing an agitation agent under pressure into the lower portion of the digester so as to agitate biomass formed by the microorganisms and to thereby improve the interaction of the biomass with the fluid during use of the digester.

12. A digester according to claim 11, characterized in that gas collection means (53) is provided for removing methane from the digester, said distribution means including a conduit (60) for conveying methane from the collection means to the digester lower portion to act as said agitation agent.

13. A digester according to any one of claims 8 to 12, characterized in that the recirculation devices are together adapted to recirculate a flow of fluid that is about five times the flow of fluid through said inlet.

14. A digester according to any one of claims 8 to 13, characterized in that said digester (2) has a sloped floor (29) for the urging of microorganism biomass toward the rcirculation devices (45, 46, 47) for the transport of the biomasses to the upper portion of the digester.

15. Apparatus according to any one of claims 8 to 13, characterized by a nutrient system for selectively providing nutrients to the microorganisms to facilitate the growth thereof; a pH control system for adjusting pH of the waste water to facilitate growth of microorganisms in the digester; and a temperature control system for selectively adjusting the temperature of the waste water before entry thereof into said digester.

**Revendications**

1. Procédé pour le traitement anaérobie d'eau usée en vue d'en éliminer les matières demandant de l'oxygène par une activité de microorganismes sur ladite eau usée à l'intérieur d'un digesteur anaérobie contenant des microorganismes producteurs de méthane dans le milieu de traitement, dans lequel l'eau usée est envoyée dans le milieu de traitement (30) qui présente une surface spécifique élevée supportant une proportion des microorganismes, on fait écouler l'eau usée à travers le milieu de traitement, on recycle en continu dans le digesteur une fraction notable de l'eau usée qui est passée dans le milieu de traitement pour lui faire subir un écoulement répété à travers ledit milieu, le volume recyclé tant de une à dix fois le volume du courant affluent, et on produit un courant effluent à partir du digesteur après une élimination notable des matières demandant de l'oxygène à partir de l'eau usée par les microorganismes tandis que du méthane est produit dans le digesteur, caractérisé par le fait que l'on distribue l'eau usée (en 38) an voisinage d'une portion supérieure du milieu de traitement (30) et on lui impose un écoulement par descente à travers le milieu; on recycle directement du fond au sommet du digesteur l'eau de recyclage portant des microorganismes pour la digestion dans la partie haute du milieu de traitement, l'eau usée de recyclage étant extraite à une pluralité d'orifices de sortie séparés (45) prévus dans le fond du digesteur et recyclée à travers une pluralité de voies (47) pour être déchargée à une pluralité d'emplacements espacés dans le sommet du digesteur; que l'on soutire un courant effluent directement à partir du digesteur en tant que fraction mineure du fluide s'écoulant à l'intérieur dudit digesteur; et que l'on élimine à partir du digesteur, séparément du courant effluent, une portion du méthane produit dans le digesteur.

2. Procédé selon la revendication 1, caractérisé par le fait que le recyclage est tel que le volume recyclé est environ cinq fois le volume du courant affluent.

3. Procédé selon la revendication 1 ou 2, caractérisé par le fait que l'on élimine le méthane à partir du sommet du digesteur et que l'on renvoie une portion du méthane dans la partie inférieure du digesteur en la laissant remonter en barbotant à travers le digesteur de façon à la faire passer à travers ledit milieu de traitement.

4. Procédé selon l'une des revendications 1, 2 ou 3, caractérisé par le fait que l'on ajoute des substances nutritives à l'affluent de façon à rendre maximale la croissance desdits microorganismes; on maintient la température dans le digesteur entre environ 35°C et 54°C; et on tamponne ledit affluent pour maintenir, dans le digesteur, un pH environ situé dans l'intervalle de 6,5 à 7.

5. Procédé selon la revendication 4, caractérisé par le fait que les substances nutritives sont des sources biologiques d'alimentation en azote et phosphore, ladite température étant située dans l'intervalle de 52°C à 54°C.

6. Procédé selon l'une des revendications précédentes, caractérisé par le fait que le courant effluent est essentiellement constitué par des moûts en provenance d'une fermentation de mélasses et d'un processus de distillation.

7. Procédé selon l'une des revendications précédentes, caractérisé par le fait que l'on recueille le méthane et qu'on le soumet à une combustion de façon à obtenir de l'énergie utilisable.

8. Digesteur ayant une entrée et une sortie et apte en fonctionnement à recevoir des microorganismes supportés par un milieu de structure poreuse à surface spécifique élevée disposé dans ledit digesteur en vue d'une interaction avec un fluide s'écoulant à travers le digesteur (2) à partir d'une entrée (38) vers une sortie (74), caractérisé par le fait que l'entrée (38) est positionnée dans une partie supérieure du digesteur (2) et la sortie (74) s'ouvre à partir d'une partie inférieure du digesteur, de façon que le fluide soit forcé de s'écouler en descendant dans le digesteur pendant son utilisation; que des dispositifs de recyclage séparés (45, 46, 47) sont prévus pour recycler en continu le fluide à partir de la partie inférieure du digesteur directement à la partie supérieure de celui-ci, de façon que l'écoulement du fluide duns le digesteur pendant son utilisation s'effectue par descente dans celui-ci et contienne des microorganismes et des substances organiques demandant de l'oxygène destinés à être renvoyés dans la partie supérieure du digesteur, lesdits dispositifs de recyclage séparés étant agencés individuellement pour extraire de l'eau usée à partir d'emplacements espacés sur le fond du digesteur et la renvoyer à différents emplacements respectifs prévus sur le sommet du digesteur, l'appareil étant dimensionné et les dispositifs de recyclage conjointement adaptés en cours d'utilisation du digesteur pour recycler un débit de liquide de un à dix fois le débit du liquide affluent pénétrant dans ladite entrée.

9. Digesteur selon la revendication 8, caractérisé par le fait que chacun des dispositifs de recyclage comprend une pompe (46) ayant un conduit d'aspiration (45) communiquant avec la partie inférieure du digesteur.

10. Digesteur selon la revendication 9, caractérisé par le fait que les conduits d'aspiration (45) sont espacés de façon sensiblement égale circonférentiellement sur le pourtour de la partie inférieure du digesteur.

11. Digesteur selon la revendication 8, 9 ou 10, caractérisé par le fait qu'un organe de distribution (60) est prévu de façon à être apte à distribuer un agent d'agitation sous pression dans la partie inférieure du digesteur, afin d'agiter la biomasse formée par les microorganismes et pour améliorer par suite l'interaction de la biomasse avec le fluide pendant l'utilisation du digesteur.

12. Digesteur selon la revendication 11, caractérisé par le fait qu'un organe (53) de collecte de gaz est prévu pour éliminer le méthane à partir du digesteur, l'organe de distribution comprenant une conduite (60) pour transporter le méthane à partir de l'organe de collecte vers la partie inférieure du digesteur en vue de servir d'agent d'agitation.

13. Digesteur selon l'une des revendications 8 à 12, caractérisé par le fait que les dispositifs de recyclage sont aptes à recycler conjointement un débit de fluide qui s'élève à environ cinqe fois le débit du fluide pénétrant dans l'entrée.

14. Digesteur selon l'une des revendications 8 à 13, caractérisé par le fait que le digesteur (2) a une plancher (29) en pente pour pousser la biomasse de microorganismes en direction des dispositifs de recyclage (45, 46, 47) en vue de transport des biomasses à la partie supérieure de digesteur.

15. Appareil selon l'une des revendications 8 à 13, caractérisé par un système nutritif destiné à fournir sélectivement des substances nutritives aux microorganismes afin de faciliter leur croissance; un système de régulation du pH pour ajuster le pH de l'eau usée en vue de faciliter la croissance des microorganismes dans le digesteur; et un système de régulation de température pour ajuster sélectivement la température de l'eau usée avant l'entrée de celle-ci dans ledit digesteur.

## Patentansprüche

1. Verfahren zur anaeroben Behandlung von Abwasser zum Entfernen von sauerstoffbeanspruchendem Material daraus durch Einwirken von Mikroorganismen auf das Abwasser innerhalb eines anaeroben Digestors, der im Medium methanerzeugende Mikroorganismen enthält, bei dem das Abwasser dem Medium (30) zugeführt wird, welches eine große Oberfläche aufweist, die einen Anteil der Mikroorganismen trägt, das Abwasser durch das Medium hindurchströmen gelassen wird, ein wesentlicher Teil des durch das Medium hindurchgeschickten Abwassers ständig zurück in den Digestor zum wiederholten Durchströmen des Mediums zurückgeführt wird, wobei das rezirkulierte Volumen zwischen dem Einfachen und dem Zehnfachen des Zuströmvolumens liegt, und eine Abflußstrom vom Digestor nach weitgehendem Entfernen des sauerstoffbeanspruchenden Materials vom Abwasser durch die Mikroorganismen unter Methanerzeugung im Digestor erzeugt wird, dadurch gekennzeichnet, daß das Abwasser (bei 38) nahe eines oberen Bereiches des Mediums (30) verteilt wird und nach unten durch das Medium hindurchgeleitet wird; das rückgeführte Wasser direkt vom Boden zum oberen Ende des Digestors rezirkuliert wird und Mikroorganismen zur Digestion zum oberen Ende des Mediums trägt, wobei das Rückführungs-Abwasser an einer Mehrzahl von getrennten Auslässen (45) am Boden des Digestors abgezogen und durch eine Mehrzahl von Kanälen (47) zur Ausströmung an einer Mehrzahl von voneinander entfernten Stellen in den oberen Bereich des Digestors rezirkuliert wird; daß ein Ausflußstrom direkt vom Digestor als ein kleinerer Teil des innerhalb des Digestors strömenden Fluids abgezogen wird; und daß ein Teil des im Digestor erzeugten Methans getrennt von Ausflußstrom abgezogen wird.

2. Verfahren nach Anspruch 1, dadurch gekennzeichnet, daß die Rückführung derart ist, daß das rückgeführte Volumen etwa dem Fünffachen des Einströmvolumens entspricht.

3. Verfahren nach Anspruch 1 oder 2, dadurch gekennzeichnet, daß das Methan von dem oberen Bereich des Digestors abgezogen wird und ein Teil des Methans zum unteren Bereich des Digestors rückgeführt und durch den Digestor derart, daß es durch das Medium hindurchtritt, hindurchperlen gelassen wird.

4. Verfahren nach Anspruch 1, 2 oder 3, dadurch gekennzeichnet, daß dem Zustrom Nährstoffe zum Maximieren des Wachstums der Mikroorganismen hinzugefügt werden; die Temperatur innerhalb des Digestors zwischen etwa 35°C und 54°C gehalten wird; und der Zustrom zur Aufrechterhaltung eines pH-Wertes etwa innerhalb des Bereichs von 6,5 bis 7 im Digestor gepuffert wird.

5. Verfahren nach Anspruch 4, dadurch gekennzeichnet, daß die Nährstoffe biologische Lieferanten von Stickstoff und Phosphor sind und die Temperatur innerhalb des Bereichs von 52 bis 54°C liegt.

6. Verfahren nach einem der vorhergehenden Ansprüche, dadurch gekennzeichnet, daß der

Ausflußstrom im wesentlichen Schlempe von einem Melasse-Fermentations- und -destillationsverfahren ist.

7. Verfahren nach einem der vorhergehenden Ansprüche, dadurch gekennzeichnet, daß das Methan gesammelt und zur Erzeugung verwertbarer Energie verbrannt wird.

8. Digestor mit einem Zufluß und einem Abfluß und einer Ausbildung derart, daß er im Betrieb von einem darin vorgesehenen Medium von porösem Aufbau und großer Oberfläche getragene Mikroorganismen zur Wechselwirkung mit einem durch den Digestor (2) von einem Zufluß (38) zu einem Abfluß (74) strömenden Fluid aufweist, dadurch gekennzeichnet, daß der Zufluß (38) in einem oberen Bereich des Digestors (2) angeordnet ist und der Abfluß (74) von einem unteren Bereich des Digestors derart mündet, daß während des Betriebes des Digestors das Fluid zu einer Abwärtsströmung durch den Digestor gezwungen wird; daß getrennte Rezirkulationsvorrichtungen (45, 46, 47) zum ständigen Rückführen von Fluid vom unteren Bereich des Digestors direkt zu dessen oberem Bereich derart, daß das Fluid im Digestor während seines Betriebs durch diesen hindurch nach unten strömt und Mikroorganismen sowie sauerstoffbeanspruchendes organisches Material, die in den oberen Bereich des Digestors rückzuführen sind, enthält, vorgesehen sind, wobei die getrennten Rezirkulationsvorrichtungen so einzeln angeordnet sind, daß sie Abwasser von voneinander entfernten Stellen am Boden des Digestors abziehen und dieses zu entsprechend verschiedenen Stellen an der Oberseite des Digestors zurückführen, wobei die Vorrichtung so ausgelegt ist, daß im Betrieb des Digestors die Rezirkulationsvorrichtungen zusammengenommen eine Flüssigkeitsströmung von zwischen dem Einfachen und dem Zehnfachen der durch den Zufluß zuströmenden Flüssigkeit rezirkulieren.

9. Digestor nach Anspruch 8, dadurch gekenn-zeichnet, daß jedes der Rezirkulations-vorrichtungen eine Pumpe (46) mit einer mit dem unteren Bereich des Digestors in Verbindung stehenden Saugleitung (45) aufweist.

10. Digestor nach Anspruch 9, dadurch gekennzeichnet, daß die Saugleitungen (45) um den unteren Bereich des Digestors herum umfangsmäßig in im wesentlichen gleichen Abständen angeordnet sind.

11. Digestor nach Anspruch 8, 9 oder 10, dadurch gekennzeichnet, daß eine Verteilvorrichtung (60) zum Verteilen eines Agitationsmittels unter druck in den unteren Bereich des Digestors, um von den Mikroorganismen gebildete Biomasse aufzurühren und dadurch die Wechselwirkung der Biomasse mit dem Fluid während des Betriebs des Digestors zu verbessern, vorgesehen ist.

12. Digestor nach Anspruch 11, dadurch gekennzeichnet, daß eine Gassammelvorrichtung (53) zum Abziehen von Methan vom Digestor vorgesehen ist, wobei die Verteilvorrichtung eine Leitung (60) zum Transport von Methan von der Sammelvorrichtung zum unteren Bereich des Digestors, damit das Methan als das Agitationsmittel wirkt, aufweist.

13. Digestor nach einem der Ansprüche 8 bis 12, dadurch gekennzeichnet, daß die Rezirkulationsvorrichtungen zusammen ausgebildet sind, eine Fluidströmung von etwa dem Fünffachen der Fluidströmung durch den Zufluß rückzuführen.

14. Digestor nach einem der Ansprüche 8 bis 13, dadurch gekennzeichnet, daß der Digestor (2) einen geneigten Boden (29) zum Drängen der Mikroorganismus-Biomasse zu den Rezirkulationsvorrichtungen (45, 46, 47) zum Transport der Biomassen zum oberen Bereich des Digestors aufweist.

15. Vorrichtung nach einem der Ansprüche 8 bis 13, gekennzeichnet durch ein Nährstoffsystem zum wahlweisen Zuführen von Nährstoffen zu den Mikroorganismen, um deren Wachstum zu erleichtern; ein pH-Steuersystem zum Einstellen des pH-Wertes des Abwassers zum Erleichtern des Wachstums der Mikroorganismen im Digestor; und ein Temperatur-Steuersystem zum wahlweisen Einstellen der Temperatur des Abwassers vor dessen Eintritt in den Digestor.

Fig.1.

0 048 148

Fig.2.

Fig.6.

Fig.3.

Fig.5.

0 048 148

Fig.4.